# EUROPEAN PATENT SPECIFICATION

(11) **EP 2 026 666 B1**
(45) Date of publication and mention of the grant of the patent: **10.06.2015**
(21) Application number: 07730088.7
(22) Date of filing: 12.06.2007
(51) Int. Cl.: A23L 1/29, A61K 36/27

(54) **UNIT SERVING APPETITE SUPPRESSANT COMPOSITIONS WITH STEROIDAL GLYCOSIDES**
STEROIDAL-GLYCOSIDE ENTHALTENDE ESSBARE APPETITZÜGELNDE PRODUKTE
COMPOSITIONS COMESTIBLES SUPPRIMANT L'APPETIT CONTENANT DES GLYCOSIDES STEROÏDIQUES

(30) Priority: 13.06.2006 EP 06115404
(43) Date of publication of application: 25.02.2009
(73) Proprietor: CSIR, Pretoria (ZA)
(72) Inventor: ABRAHAMSE, Salomon, Leendert, 3133 AT Vlaardingen (NL); POVEY, Kevin, John, Purfleet, Essex RM19 1SD (GB); REES, Daryl, David, Godmanchester, Cambridgeshire PE29 2HY (GB)
(74) Representative: Brown, David Leslie
(86) International application number: PCT/EP2007/055764
(87) International publication number: WO 2007/144347

(56) References cited:
- EP-A- 1 166 792
- WO-A-03/041727
- US-A- 3 966 918
- US-A1- 2005 266 137
- US-A1- 2005 276 839
- US-A1- 2006 083 795
- "Commision Directive 96/8/EC of 26 February 1996 on foods intended for use in energy-restricted diets for weigth reduction" INTERNET CITATION, [Online] 26 February 1996 (1996-02-26), XP002310347 Retrieved from the Internet: URL:http://europa.eu.int/comm/food/food/la bellingnutrition/weight/df09_en.pd> [retrieved on 2004-12-06]
- SCHONER W: "Endogenous cardiac glycosides, a new class of steroid hormones", EUROPEAN JOURNAL OF BIOCHEMISTRY, BLACKWELL PUBLISHING, BERLIN, DE, vol. 269, 1 January 2002 (2002-01-01), pages 2440-2448, XP002367830, ISSN: 0014-2956, DOI: 10.1046/J.1432-1033.2002.02911.X

## Description

### TECHNICAL FIELD

The present invention relates generally to appetite suppressant compositions. More particularly, it relates to unit serving appetite suppressant compositions comprising steroidal glycosides from plants of *Hoodia* genus.

### BACKGROUND OF THE INVENTION

In the Western world, but also in other countries where a so-called Western diet is adopted, the incidence of being overweight and obese has drastically increased over the last decades.

Since obesity and being overweight are generally known to be associated with a variety of diseases such as heart disease, type 2 diabetes, hypertension and arthrosclerosis, this increase is a major health concern for the medical world and for individuals alike. Furthermore, people who are overweight may consider themselves as unattractive, which leads to a clearly reduced feeling of well-being.

This has led to an increasing interest by consumers in their health and has created a demand for products that help to reduce or control daily caloric intake and/or control body weight and/or bodily appearance.

Several solutions have been proposed to help individuals to control their weight. Among these solutions is the use of drugs e.g. to suppress the activity of enzymes in the digestive system. However, the use of drugs is often not preferred unless strictly required for medical purposes.

Another proposed solution is to prescribe the individuals a specific diet, for example, a diet with a restricted caloric intake per day.

Meal replacement products have also been proposed as part of a healthy diet in order to control or reduce body weight. For example, US 5688547 discloses a nutritional meal replacement composition comprising dietary fiber, protein and a cellulose gum and gel. Meal replacement products are generally products that are intended to be consumed as a single-serving food product, such as a bar, drink etc to replace one or two meals per day. Commercial meal replacement products include, for instance, the Slim-Fast® brand (http://www.slim-fast.com). See also US 2005/0266137 (Eppler) and US 2005/018019 (Palmer).

Unfortunately, a common problem with meal replacements and other conventional diets is that people remain hungry and this is a key reason people fail to stick to the diet and, therefore, fail to reduce calorie intake and lose weight. Thus, overall calories reductions are less than ideal and nutrition is overcompensated. These factors may render it difficult for the individual to adhere to the plan or it may make it and/or the products used therein less appealing to consumers.

European Patent EP-A-994 655 discloses appetite suppressant compositions comprising an emulsion containing palm oil, oat oil and water.

Extracts from plants of the *Hoodia* genus (formerly the *Hoodia* and *Trichocaulon* genera) which contain steroidal glycosides have also been shown to have an appetite suppressant activity. See US Patent 6,376,657. US 2005/0202103 (Rajendran et al.) discloses steroidal glycosides obtainable from *Carolluma,* another genus of plants in *Asclepidaceae* family. US 7,008,648 (Corley et al.) discloses appetite suppression compositions containing steroidal glycosides obtainable from *Stapelia* and *Orbea* plants, additional plants within *Asclepidoceae* family. US 2005/0276839 (Rifkin) and US 2006/0083795 (Shatkina) disclose appetite suppressant compositions containing *Hoodia gordonii.*

The problems with existing appetite suppressant products are that
1) they do not reduce calorie intake and do not provide weight loss because the appetite suppressant content is too low; and/or
2) they are open to consumer abuse where lots of appetite suppressant can be ingested without any calorie or nutrition provided or where too many products and, therefore, too many calories are ingested; and/or
3) formulation differences can alter the bioavailability of the active appetite suppressant and its effect.

For these reasons, it is unclear how to set the correct level of appetite suppressant and total calorie (total energy) in different product formats. Thus, incorporating an appetite suppressant into a diet creates new challenges for the product developer to provide the correct conditions to achieve weight loss through appetite suppression, yet also maintain an adequate level of nutrition, (not too little and not too much).

A particular challenge exists for formulating unit serving appetite suppressant products - since many a day may be consumed (due to relatively small size and typically a satisfying snack-like taste and experience), potentially delivering relatively high amounts of both the appetite suppressant and the calorie intake. It is important to design a unit serving product that provides energy intake reduction, even when relatively high number of servings are consumed, while also ensuring that energy intake reduction and sufficient nutrition is achieved from even a single daily serving.

Accordingly, there is still a need for appetite suppressant unit serving products, which are inherently nourishing providing at least a minimum of energy intake, yet also suppress appetite and maintain appetite suppression even at increased number of servings. In particular, there is a need for such appetite suppressant products comprising *Hoodia* steroidal glycosides.

### SUMMARY OF THE INVENTION

The present invention is defined in and by the appended claims. We have now surprisingly found that these and other objects of the invention may be achieved by an appetite suppressant unit serving product in the form of a bar having a weight from 20 to 100 g, or a drink having a volume from 80 to 600mL, the product comprising:
(a) from 50 to 2000 milligrams of a steroidal glycoside;
(b) from 200 to 2,000 kilojoules total energy;
(c) the steroidal glycoside to total energy ratio of from 1: 0.5 to 1 : 10 mg/kJ;
(d) an essential mineral selected from the group consisting of phosphorus, iron, zinc, copper, selenium, magnesium, manganese, molybdenum, chromium and mixtures thereof.

The invention is based at least in part on the discovery that the inventive product ensures that the appetite suppressant cannot be ingested (consumed) without the consumer getting a minimum daily level of energy intake, including essential minerals and preferably in the form of a healthy balance of proteins, fats and carbohydrates to total energy content; and furthermore that a relatively high number of products may be ingested while maintaining daily energy intake reduction and also preferably in the optimum ratio of steroidal glycosides to protein. The inventive product provides healthy weight loss, yet avoids too rapid weight loss or insufficient energy intake by anorexics or extreme dieters.

In a second aspect, the invention relates to a method of using such compositions for suppressing appetite and/or attaining daily energy reduction intake.

The average typical daily energy intake for an adult is around 10,000 kJ for women and around 13,000 kJ for man. It increases steadily with increasing BMI (body mass index) to up to 14,000 kJ for women and 18,000 kJ for men.

The method according to the invention may attain energy intake reductions of from 750 to 4500 kJ per day, more preferably from 1000 to 4500 kJ per day, most preferably from 2000 to 4000 kJ per day while maintaining adequate nutrition by balancing the longer term (typically 14 days or longer) effect of steroidal glycosides with the intake of essential good nutrition. This reduction in daily energy intake is achieved even when a relatively high number of the unit servings are consumed per day, in addition to the regular daily energy intake.

The inventive products prevent consumer abuse of undernourishment which is a typical situation with a diet based on appetite suppressants in pills and/or very low calorie supplements.

The inventive products also promote consumer compliance by providing nutritious tasty products with sufficient nourishment.

The inventive method prevents consumers overeating or under-eating by correctly balancing desire to eat with nutrition provided.

### DETAILED DESCRIPTION OF THE INVENTION

Except in the operating and comparative examples, or where otherwise explicitly indicated, all numbers in this description indicating amounts of material or conditions of reaction, physical properties of materials and/or use are to be understood as modified by the word "about." All amounts are by weight of the final composition, unless otherwise specified.

It should be noted that in specifying any range of concentration or amount, any particular upper concentration can be associated with any particular lower concentration or amount. The amounts of all ingredients besides the steroidal glycoside, including proteins, fats, carbohydrates, etc. are meant herein to be those that are delivered through ingredients other than the steroidal glycoside source (e.g., extract, plant, etc.).

For the avoidance of doubt the word "comprising" is intended to mean "including" but not necessarily "consisting of" or "composed of." In other words, the listed steps or options need not be exhaustive.

"Unit serving" as used herein means an individual serving, designed for consumption at one time and, preferably individually packaged. It is sometimes also described herein as "single serving."

A "drink" as used herein includes a dairy, fruit or vegetable based drink in either a sweet or savory base.

A "bar" as used herein includes snack or meal bars and also any small hand held and/or bite sized food product (e.g., cookies, muffins).

### STEROIDAL GLYCOSIDE

The inventive edible appetite suppressant compositions comprise a steroidal glycoside. "Steroidal glycoside" as used herein means a steroid (four fused rings), further comprising at Least one side group substitution which is a glycoside (a molecule in which a sugar group is bonded through its anomeric carbon to another group via an O-glycosidic bond), preferably a deoxy or di-deoxy glycoside.

The steroidal glycoside is obtainable from *Hoodia* plants. This method is, disclosed in the US Patent 6,376,657. Other methods of preparing steroidal glycosides are also acceptable.

Most preferably, the steroidal glycoside is delivered to the inventive compositions in the form of an extract from the *Hoodia* plants.

Suitable plants include but are not limited to *Trichocaulon piliferum, Trichocaulon officinale*, *Hoodia currorii, Hoodia gordonii, Hoodia lugardii* and mixtures thereof. More preferably, the plant extract is from *Hoodia gordonii.*

Suitable steroidal glycoside compounds include but are not limited to the general structural formula (1): 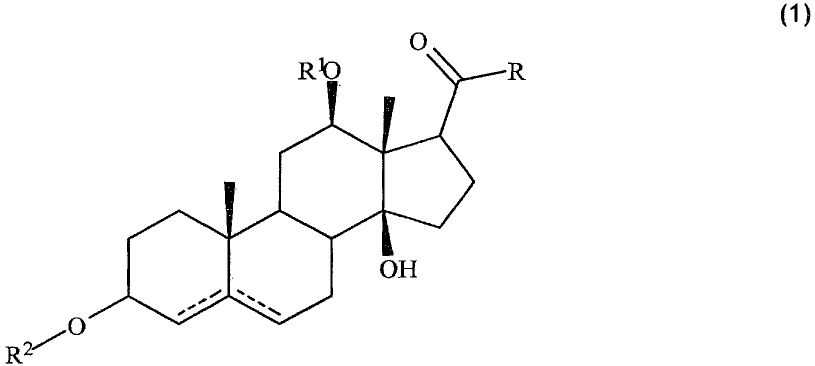 wherein
R = alkyl;
R¹ = H, alkyl, tiglyol, benzoyl or any other organic ester group;
R² = H or one or more 6-deoxy carbohydrates, or one or more 2,6-dideoxy carbohydrates, or glucose radical, or combinations thereof; and wherein the broken lines indicate the optional presence of a further bond between carbon atoms C4 and C5 or between carbon atoms C5 and C6.

Particularly preferred steroidal glycosides are analogs of Compound of Formula 1, extracted from the plants, including Compounds of Formula (2) through Formula (8), and mixtures thereof, obtainable from the *Hoodia* plants.

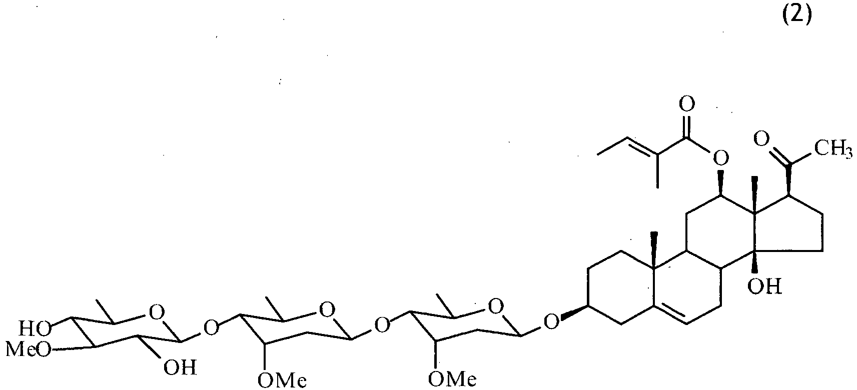

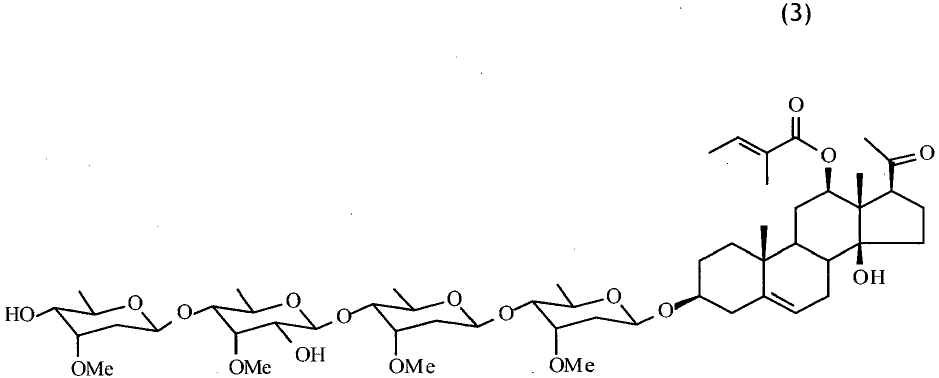

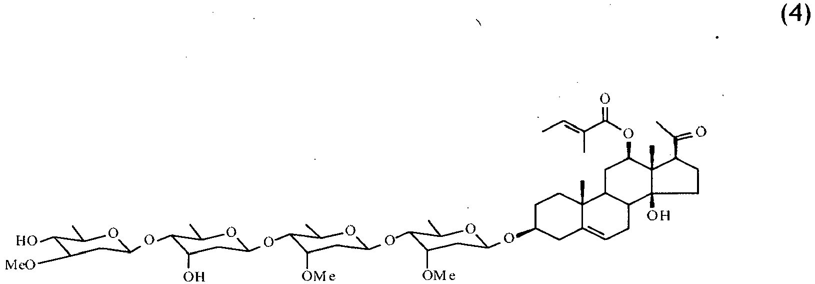

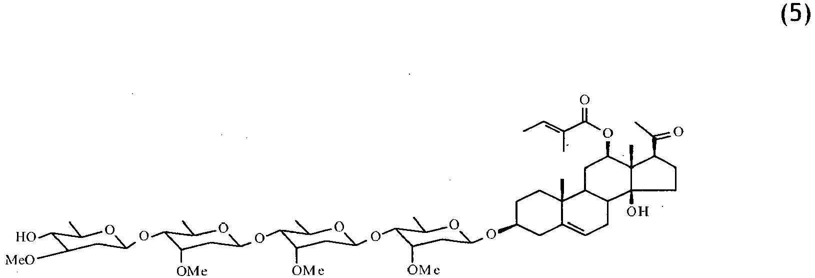

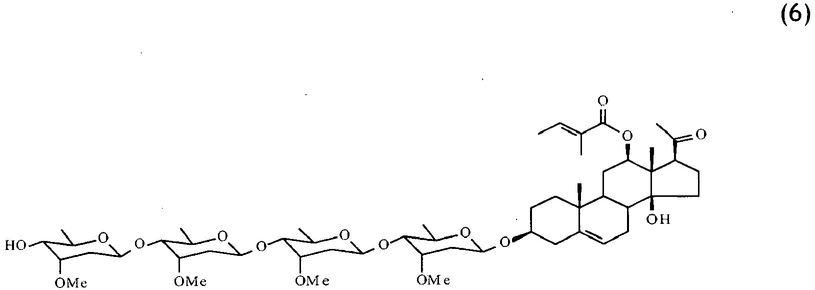

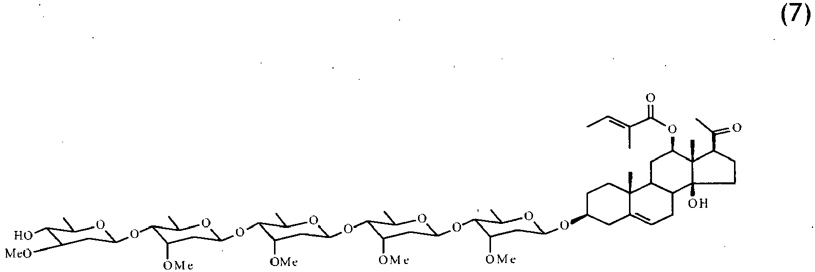

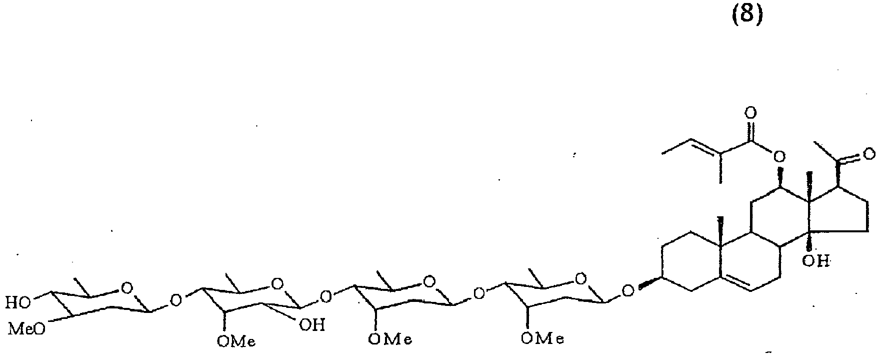

Other steroidal glycosides not specifically mentioned herein may be included in the inventive product. It will be understood that the invention also encompasses isomers, derivatives, salts, esters and analogs of the steroidal glycosides (preferably, biologically active) and mixtures thereof.

The product according to the invention comprises from 50 to 2000 milligrams ("mg"), preferably from 70 to 1500 mg, more preferably from 80 to 1200 mg, most preferably from 90 to 1100 mg, optimally from 100 to 1000 mg of a steroidal glycoside, in order to provide the optimum and consistent appetite suppressant response among the population from unit serving bars or drinks.

When an extract is used, the extract typically comprises at least 10%, by weight of the extract, of steroidal glycosides, preferably at least 20%, most preferably at least 60%, and optimally at least 70%. US Patent 6,376,657, describes the preparation of a suitable extract comprising steroidal glycosides from the genus *Hoodia,* said steroidal glycoside having appetite suppressant activity. The solvents specifically mentioned to perform the extraction are one or more of methylene chloride (dichloromethane), water, methanol, hexane, ethyl acetate or mixtures thereof. An alternative method to obtain an extract is disclosed by separating plant sap from the plant solid material. "Extract" as used herein includes liquid, solid or spray-dried forms of extracts, sap, which also may be purified, partially purified, concentrated and/or fractionated. Other methods of extracting a steroidal glycoside from plants are also suitable.

Besides the extract, the steroidal glycoside may be incorporated in the form of otherwise concentrated preparation, e.g. a dried plant or otherwise concentrated plant preparation, which preferably contains the same steroidal glycoside amounts as described above with respect to the extract. Steroidal glycoside concentrations may be determined using high performance liquid chromatography (HPLC) with UV detection after extraction or dissolution.

In case of dried plant material approximately 5 g of material may be refluxed with approx. 80 ml of boiling methanol for 1 hour. The resulting extract is filtered and the solid material is washed with methanol. The combined filtrate and washing are transferred to a 100 ml flask and made to volume with methanol. 1 ml of the filtrate is evaporated to dryness and reconstituted in 1 ml acetonitrile/water (50/50 v/v).

In case of extracts approximately 20 mg of the material may be dissolved in 50 ml of methanol by sonication for 10 minutes. After filtration 1 ml of the filtrate is evaporated to dryness and reconstituted in 1 ml acetonitrile/water (50/50 v/v).

Steroidal glycosides may be measured by LC-UV at 220 nm. To this end 20 µl of the extracts may be injected onto a Zorbax RX-C8 analytical column of 250 x 4.6 mm packed with 5 µm particles and equipped with a Zorbax RX-C8 guard column of 12.5 x 4.6 mm packed with the same stationary phase. The column system is held at 40°C. Gradient elution is performed starting at 41.2% acetonitrile/methanol (85/15 v/v) and 58.8% water/methanol (85/15 v/v) at a flow rate of 1 ml/min. Initial conditions are held for 10 minutes before being linearly increased to 88.2% acetonitrile/methanol (85/15 v/v) and 11.8% water/methanol (85/15 v/v) over 30 minutes. After a final hold of 5 minutes the system is re-equilibrated to the starting conditions.

Compound of Formula 2 (as hereinabove) of any known purity (95% was used in this case) may be used for calibration. Compound 2 may be isolated from an extract of dried *Hoodia* gordonii using preparative liquid chromatography or may be synthesized (see e.g. US Patent 6,376,657). A stock solution at 100 µg/ml may be prepared in acetonitrile/water (1/1 v/v) and further dilutions are prepared to yield additional calibration standards at 75, 50, 20, 10 and 5 µg/mL UV response at 220 nm may be used for quantification against the Compound 2 calibration line. Relative response factors based on molecular weight are used to quantify the steroidal glycosides against the Compound 2 calibration line. Steroidal glycosides are defined as all peaks eluting after 15 min that are not present in the blank acetonitrile/water (1/1 v/v) sample. For the compounds of Formula 2-8 the specific relative retention times and response factors, are summarized in Table 1.

**TABLE 1**

| Relative retention times and response factors of some steroidal glycosides | | |
|---|---|---|
| Compound | Relative retention time vs. Compound 2 | Response factor vs. Compound 2 |
| formula 2 | 1.000 | 1.000 |
| formula 8 | 1.066 | 1.164 |
| formula 3 | 1.128 | 1.164 |
| formula 4 | 1.191 | 1.130 |
| formula 5 | 1.292 | 1.146 |
| formula 6 | 1.328 | 1.146 |
| formula 7 | 1.399 | 1.309 |

The other steroidal glycosides peaks eluting after 15 minutes have a response factor of 1.081 vs. Compound 2.

### PRODUCT FORMAT

The product format of the edible appetite suppressant product is a unit serving drink or bar. A bar weight is from 20 to 100 g, preferably from 25 to 75 g, most preferably from 25 to 60 g. A drink has a volume of 80 to 600 ml, preferably 90 to 400 ml, most preferably from 100 to 350 ml. Preferably each unit serving is separately packaged and includes instructions, in particular recommendation of the number of the particular unit serving to be consumed per day.

### ENERGY CONTENT

The product of the invention has the energy content of from 200 to 2000 kiloJouLes("kJ"), preferably from 250 to 1900 kJ, more preferably from 300 to 1800 kJ, most preferably from 350 to 1500 kJ, optimally from 400 to 1200 kJ, in order to deliver optimum weight control and nutritional benefits. The energy in the inventive product is delivered through healthy balance of proteins, fats and carbohydrates, as described below.

### RATIO OF STEROIDAL GLYCOSIDE TO ENERGY CONTENT

According to the present invention, the appetite suppressing unit serving products are formulated to include steroidal glycosides in a defined ratio to the total energy content, such as to achieve the reduction in daily energy intake, yet provide sufficient nourishment, even when product is overused.

The ratio of steroidal glycoside amount to total energy content is in the range of from 1:0.5 to 1:10 (mg/kJ), preferably from 1:0.7 to 1:8, most preferably from 1:0.9 to 1:6, optimally from 1:1 to 1:5.

### ESSENTIAL MINERALS

In particular, the inventive compositions include essential minerals selected from the group consisting of phosphorus, iron, zinc, copper, selenium, magnesium, manganese, molybdenum and chromium, especially iron, phosphorous and zinc, and most especially iron as these are considered essential for the nutrition and the lack of one or more of these, and especially iron, in sufficient amounts may Lead to health problems. The minerals described in this paragraph may be added minerals rather than the trace amounts that may be present in various raw ingredients.

The preferred ingredients to deliver these minerals include but are not limited to magnesium oxide, ferrous sulfate, ferrous lactate, ferrous fumarate, ferric pyrophosphate, ferric orthophosphate, carbonyl iron, electrolytic iron, NaFeEDTA, ferric orthophosphate, zinc oxide, zinc gluconate, chromium chloride, sodium selenate, manganese sulfate and mixtures thereof.

The various minerals may be included in the inventive products in the amounts as follows:

| | Units | Range (per unit serving) | | Preferred range(per unit serving) | |
|---|---|---|---|---|---|
| Mineral | | Low | High | Low | High |
| Phosphorous | mg | 100 | 1000 | 300 | 500 |
| Iron | mg | 1 | 10 | 3 | 7 |
| Zinc | mg | 1 | 7 | 2 | 6 |
| Magnesium | µg | 20 | 200 | 80 | 150 |
| Selenium | µg | 3 | 40 | 7 | 25 |
| Chromium | µg | 5 | 50 | 10 | 42 |
| Molybdenum | µg | 3 | 40 | 7.5 | 27 |
| Manganese | mg | 0.05 | 6 | 0.2 | 1 |
| Copper | mg | 0.1 | 2 | 0.2 | 0.6 |

In the preferred embodiment of the invention, the range of steroidal glycosides to iron is from 25:1 to 500:1 (mg steroidal glycosides: mg iron), preferably from 40:1 to 400:1, most preferably 50:1 to 300:1.

### PROTEINS, FATS, CARBOHYDRATES

The energy content in the inventive products is delivered through a healthy balance of proteins, fats, and carbohydrates.

According to the present invention, it is particularly important to maintain the protein amount, in cases where the inventive products are consumed in lieu of regular meals. Suitable proteins include but are not limited to milk, skimmed milk, fat free milk, condensed milk, fermented milk, cream, whey, yoghurt, cheese, egg, buttermilk, milk powder, buttermilk powder, cream powder, whey powder, yoghurt powder, cheese powder, egg powder, calcium and sodium caseinates, lactose free dairy proteins, soy proteins, isolated soy proteins, vegetable proteins, meat and fish derived proteins, gelatin, albumin powder, and mixtures thereof.

In the most preferred embodiment of the invention, an optimum balance between the amount of proteins and steroidal glycoside amount is maintained, in the range of from 0.5:1 to 200:1 (mg protein: mg steroidal glycoside), preferably from 2:1 to 100:1, most preferably from 5:1 to 75:1.

Suitable carbohydrates include but are not limited to potato, pasta, wheat, corn, soy fiber, fruit fiber (e.g. apple and orange),sucrose, fructose, dextrose, lactose, maltodextrins, honey, corn syrup, oligofructose, starches (e.g potato starch, corn starch, rice starch), modified starches, fruit juice, concentrated fruit juice, flours (e.g wheat, corn and rice), gums (e.g. xanthan gum, guar gum, gum arabic, locust bean gum, celluloses (e.g sodium carboxy methyl cellulose, microcrystalline cellulose, powdered cellulose), carageenan, potassium carageenan, alginates (e.g sodium and potassium alginates), gelatin, pectin and mixtures thereof.

It should be noted that some or all of the sugar may be replaced by artificial sweeteners, or artificial sweeteners may be present in addition to sugars. Artificial sweeteners include but are not limited to aspartame, cyclamates (e.g. Sodium cyclamate), acesulfame K, sucralose, saccharin, invert sugar, maltose sugar, sugar alcohols (e.g maltitol, sorbitol)and mixtures thereof.

Suitable fats include but are not limited to saturated and unsaturated fats and oils, for instance sunflower oil, high oleic sunflower oil, canola, cottonseed oil, corn/maize oil, rapeseed oil, olive oil, soybean oil, palm oil, palm kernel oil, coconut, fish oil, linseed oil, peanut/groundnut oil, safflower oil, sesame oil, butter, lard, cocoa butter, mono and diglycerides and mixtures thereof.

The sources of oils and fats can also be hardened (e.g. by hydrogenation) or fractionated (e.g. via solvents) and these can be mixed with other oils or fats.

In order to optimise the health value of the inventive products, at least some of the fat, generally from 10 to 80%, preferably from 30 to 50%, by weight of the total fat, may be present as unsaturated or polyunsaturated oil, in particular oils which contain linoleic (e.g sunflower, soybean, corn, Linola^{tm} or rapeseed)or linolenic acid (e.g linseed) and mixtures thereof. Ideally the product should deliver at least 1g per day of linoleic and/or linolenic acid, preferably from an unsaturated fat source.

To minimise the potential harmful effects, the inventive products may be, substantially free of trans fat, i.e. contain less then 0.5% of trans fat, preferably less then 0.1%, most preferably less than 0.05% and optimally 0% trans fat, by weight of the product.

The general and preferred ranges for the relative amounts of energy from fat and protein in embodiments of the inventive product is as follows:

| Ranges | % energy from fat | % energy from protein |
|---|---|---|
| General | 0.5-55 | 3-50 |
| Preferred | 2-45 | 5-45 |
| Most Preferred | 5-40 | 10-40 |
| Optimal | 10-30 | 10-35 |

Typically, carbohydrates deliver the balance of the energy requirement. Generally, this means according to the present invention, that carbohydrates may contribute between 10 and 75% energy in the product. At the preferred, most preferred and optimal ranges, the best nutritional benefit may be delivered at an optimum cost.

The especially preferred unit serving drink according to the invention has the following composition, in addition to the steroidal glycoside:
75-95 wt% (preferably 80-90 wt%) moisture
0.5-10 wt% (preferably 1-7 wt%) protein
0.5-6 wt% (preferably 0.6-5 wt%) fat
3-20 wt% (preferably 4-15 wt%) carbohydrate and
up to 8 wt% (preferably 1-6 wt%) minor components

The especially preferred unit serving bar according to the invention has the following composition, in addition to the steroidal glycoside:
3-30 wt% (preferably 5-25 wt%) moisture
3-30 wt% (preferably 5-25 wt%) protein
3-30 wt% (preferably 5-25 wt%) fat
35-80 wt% (preferably 40-75 wt%) carbohydrate and
up to 12 wt% (preferably 1-10 wt%) minor components

### METHOD OF USING

The inventive product is used for suppressing appetite in humans and/or as a product for use in a method of controlling obesity in a human, while at the same time providing at least a minimum nutrition and a balanced intake of essential minerals, proteins, fats, and carbohydrates.

Generally, at least one inventive product should be ingested per day, typically from 1 to 5 per day (per 24 hours), until reaching the desired weight, and then continuing with this regime to maintain the desired weight. Most preferably, from 1 to 3 products are consumed per day for optimum effect. Most preferably, the inventive products are consumed for at least 14 consecutive days.

Preferably, the inventive products are consumed in addition to the regular energy intake, with a total daily amount of steroidal glycosides from the unit serving products in the range of from 50 mg to 4000 mg, preferably from 70 mg to 2000 mg, most preferably from 90 mg to 1500 mg. The total daily energy intake from the unit serving products is in the range of from 200 kJ to 5000 kJ, preferably from 400kJ to 2000 kJ, most preferably from 600 kJ to 1500 kJ.

Generally, by using the products of the invention and/or following the inventive dietary regimen weight loss of 0.1 kg to 3 kg, preferably from 0.2 kg to 2 kg, most preferably from 0.5 kg to 1 kg per week may be achieved.

The particular virtue of the invention is that even at the overuse of the product, sufficient energy levels are provided, to ensure an adequate energy intake for healthy dieting. Furthermore, the right balance between the amount of steroidal glycoside extract and the energy content of the unit serving can be attained. The inventive products are designed to give good nutrition while providing effective reduction in energy to enable steady weight loss. The ratios of steroidal glycoside to total energy, to the essential minerals (especially iron) and to proteins are crucial when balancing the opposing aims of providing weight loss and also the required proper nutrition.

### OPTIONAL INGREDIENTS

The inventive composition preferably includes additional nutrients, vitamins and additional minerals to deliver healthy nutrition, despite the appetite suppression. Suitable vitamins and minerals, include but are not limited to Vitamin A, Vitamin D, Vitamin E, Vitamin C, Thiamin, Riboflavin, Niacin, Vitamin B6, folate, Vitamin B12, Biotin, Pantothenic acid, Calcium, Potassium, Sodium, iodine, vitamin K, and mixtures thereof.

The preferred ingredients to deliver vitamins and minerals include but are not limited to potassium phosphate, calcium phosphate, magnesium oxide, magnesium phosphate ascorbic acid, sodium ascorbate, vitamin E acetate, niacinamide, ferric orthophosphate, calcium pantothenate, zinc oxide, zinc gluconate, vitamin A palmitate, pyridoxine hydrochloride, riboflavin, thiamin mononitrate, biotin, folic acid, chromium chloride, potassium iodide, sodium molybdate, sodium selenate, phytonadone (vitamin K), cholecalciferol (vitamin D3), cyanocobalamin (vitamin b12), manganese sulfate and mixtures thereof. Preferably, the inventive product contains at least 10% or more of the recommended daily amount ("RDA") of the vitamins and minerals.

Another especially preferred optional ingredient is fiber. Suitable fiber sources include but are not limited to: inulin /oligofructose, gum arabic, cellulose, cellulose gum, wheat fiber (nutriose), fruit pulp/fiber, pectin, guar gum. Fiber is included generally in an amount of from 0.5-10 g per product, preferably 0.8 to 8, most preferably from 1 to 5.
The inventive products may further include meat, fish, meat and fish extracts, fruit, dried fruit, fruit concentrates, fruit extracts, fruit juices, tea (e.g. green tea) vegetables, vegetable extracts and concentrates, nuts, nut extracts, chocolate, bread, vinegar, salt, pepper, cocoa powder, herbs (e.g. parsley), herb extracts, spices (e.g. cinnamon), spice extracts, emulsifiers, acidity regulators (e.g. phosphoric, malic, citric,tartaric acids and salts thereof), flavonoids, preservatives (e.g. lactic acid, EDTA, tocopherols, sodium benzoate), colors (e.g. beta carotene, lycopene, caramel, carmine red), fibers (e.g. soy), leavening agents (e.g., sodium bicarbonate), pectin, citric acid, yeast, salt, glycerine, and mixtures thereof.

The preferred inventive products are substantially free of cholesterol, i.e. the products comprise less than 10 mg of cholesterol, preferably no more than 5 mg per unit serving, and optimally are free of cholesterol. The preferred products include sterols and/or stanols for cholesterol lowering effects.

The preferred inventive products comprise less than 6g of sodium, preferably less than 3g, most preferably less than 1 g, optimally less than 150 mg per unit serving.

The preferred inventive products contain at least 70 mg of potassium, preferably at least 100 mg, most preferably at least 140 mg per unit serving.

### PROCESS OF PREPARING

A steroidal glycoside may be incorporated into the inventive unit serving product in the same manner as any food ingredient. Preferably, the steroidal glycoside is finely dispersed, preferably delivered in the extract from plants. The extract is prepared as described above, or by CO₂ extraction, or by any other suitable method.

All amounts, parts, ratios and percentages used herein are by weight, unless otherwise specified.

The invention will now be further illustrated in the following non-limiting examples.

### Example 1

The following appetite suppressant Muesli Bar, Yoghurt Muesli Variant, is within the scope of the invention:

| **Formulation:** | | | **Nutritional Info:** | | |
|---|---|---|---|---|---|
| **Ingredient** | **% Formula** | | Product weight as consumed: | 60g | |
| Maltitol | 4.350 | | Energy density (kJ/g) | 14.62 | |
| Glucose syrup | 8.403 | | **Energy** | **Per 100g** | **Per 60g Bar** |
| Polydextrose syrup | 13.344 | | | | |
| Inulin syrup | 5.600 | | Energy Value (kJ) | 1465 | 877 |
| Coconut oil | 1.000 | | Energy Value (kcal) | 347 | 208 |
| Mazola oil | 2.300 | | % Energy from Protein | - | 27.50 |
| Brown Sugar | 1.000 | | % Energy from Carbohydrates | - | 48.65 |
| Lecithin | 0.600 | | % Energy from Fat | - | 28.13 |
| Pectose paste | 5.000 | | Trans Fat (g) | 0.087 | 0.052 |
| Glycerine | 5.000 | | % Energy from Trans Fat | - | 0.23 |
| Date paste | 3.000 | | Linoleic Acid (g) | - | 1.42 |
| Flavourings | 0.405 | | Sodium (mg) | 390 | 230 |
| Colouring | 0.144 | | Cholesterol (mg) | N/A | N/A |
| Oatflakes | 3.706 | | **Vitamins** | **Per 100g** | **Per 60g Bar** |
| Coconut flakes | 1.900 | | | | |
| Apple Fiber | 3.900 | | Vitamin A (µg) | 380.00 | 228.00 |
| Soy Nuggets | 31.100 | | Vitamin D (µg) | 3.33 | 2.00 |
| Vitamin & Mineral Premix | 3.914 | | Vitamin E (mg) | 6.70 | 4.02 |
| Yogurt Coating | 10.000 | | Vitamin C (mg) | 30.00 | 18.00 |
| Plant Extract (80% steroidal glycosides) | 1.000 | | Thiamin (mg) | 0.83 | 0.50 |
| | | | Riboflavin (mg) | 0.81 | 0.49 |
| Water loss during manufacture | -5.666 | | Niacin (mg) | 10.30 | 6.18 |
| | | | Vitamin B6 (mg) | 1.00 | 0.60 |
| **TOTAL** | **100.000** | | Folic Acid (µg) | 140.00 | 84.00 |
| | | | Vitamin B12 (µg) | 1.47 | 0.88 |
| | | | Biotin (mg) | 0.05 | 0.03 |
| | | | Pantothenic Acid (mg) | 2.65 | 1.59 |
| | | | | | |
| | | | **Minerals** | **Per 100g** | **Per 60g Bar** |
| | | | | | |
| | | | Calcium (mg) | 368.00 | 220.80 |
| | | | Phosphorus (mg) | 534.00 | 320.40 |
| | | | Iron (mg) | 9.60 | 5.76 |
| | | | Magnesium (mg) | 90.00 | 54.00 |
| | | | Zinc (mg) | 5.70 | 3.42 |
| | | | Iodine (µg) | 70.00 | 42.00 |
| | | | Potassium (mg) | 833.34 | 500.00 |
| | | | Copper (mg) | 0.70 | 0.42 |
| | | | Selenium (µg) | 32.50 | 19.50 |
| | | | Manganese (mg) | 0.70 | 0.42 |

| | | | | | |
|---|---|---|---|---|---|
| % protein content for the bar = 23.8 % carbohydrate content for the bar = 42.2 % fat content for the bar = 10.8 | | | | | |

### Example 2

The following appetite suppressant Chicken & Mushroom Soup is within a scope of the invention:

| **Formulation:** | | | **Nutritional Info:** | | |
|---|---|---|---|---|---|
| **Ingredient** | **% Formula** | | Product weight as consumed: | 295ml | |
| Water | To 100% | | **Energy density (kJ/g)** | **2.94** | |
| Skimmed Milk Powder | 1.218 | | **Energy** | **Per 100ml** | **Per 295ml** |
| Sodium Phosphate | 0.126 | | | | |
| Corn Oil | 1.034 | | Energy Value (kJ) | 311 | 918 |
| Butter Concentrate | 0.528 | | Energy Value (kcal) | 74 | 218 |
| Wheat Flour | 2.002 | | % Energy from Protein | - | 36.70 |
| Modified Maize Starch | 1.575 | | % Energy from Carbohydrates | - | 34.68 |
| Flavourings | 0.567 | | % Energy from Fat | - | 30.14 |
| White Pepper | 0.014 | | Trans Fat (g) | N/A | N/A |
| Mace Powder | 0.007 | | % Energy from Trans Fat | - | N/A |
| Maltodextrin | 2.800 | | Linoleic Acid (g) | - | 1.69 |
| Titanium Dioxide | 0.182 | | Sodium (mg) | 360 | 1060 |
| Garlic Powder | 0.028 | | Cholesterol (mg) | N/A | N/A |
| Onion Powder | 0.028 | | **Vitamins** | **Per 100ml** | **Per 295ml** |
| Gelatine | 3.150 | | | | |
| Salt | 0.399 | | Vitamin A (µg) | 92.54 | 273.00 |
| MSG | 0.280 | | Vitamin D (µg) | 0.66 | 1.95 |
| Vitamin & Mineral | 0.858 | | Vitamin E (mg) | 1.32 | 3.90 |
| Parsley | 0.406 | | Vitamin C (mg) | 9.15 | 27.00 |
| Chicken Meat | 10.544 | | Thiamin (mg) | 0.14 | 0.40 |
| Mushrooms | 4.461 | | Riboflavin (mg) | 0.20 | 0.58 |
| Cream | 0.811 | | Niacin (mg) | 2.20 | 6.50 |
| Plant Extract (80% steroidal glycosides) | 0.330 | | Vitamin B6 (mg) | 0.18 | 0.54 |
| | | | Folic Acid (µg) | 24.41 | 72.00 |
| **TOTAL** | **100.00** | | Vitamin B12 (µg) | 0.17 | 0.50 |
| | | | Biotin (mg) | 0.002 | 0.005 |
| | | | Pantothenic Acid (mg) | 0.37 | 1.10 |
| | | | | | |
| | | | **Minerals** | **Per 100ml** | **Per 295ml** |
| | | | | | |
| | | | Calcium (mg) | 85.42 | 252.00 |
| | | | Phosphorus (mg) | 96.61 | 285.00 |
| | | | Iron (mg) | 1.97 | 5.80 |
| | | | Magnesium (mg) | 18.31 | 54.00 |
| | | | Zinc (mg) | 1.15 | 3.40 |
| | | | Iodine (µg) | 15.93 | 47.00 |
| | | | Potassium (mg) | 186.44 | 550.00 |
| | | | Copper (mg) | 0.14 | 0.40 |
| | | | Selenium (µg) | 6.71 | 19.80 |
| | | | Manganese (mg) | 0.12 | 0.36 |

| | | | | | |
|---|---|---|---|---|---|
| % protein content for the drink = 6.8 % carbohydrate content for the drink = 6.4 % fat content for the drink = 2.5 | | | | | |

### Example 3

The following appetite suppressant Strawberry Milk Drink is within the scope of the invention:

| **Formulation:** | | | **Nutritional Info:** | | |
|---|---|---|---|---|---|
| **Ingredient** | **% Formula** | | Product weight as consumed: | 325ml | |
| Skim Milk | 75.300 | | Energy density (kJ/g) | 2.65 | |
| Water | To 100% | | **Energy** | **Per 100ml** | **Per 325ml** |
| Sucrose | 6.600 | | | | |
| Gum Arabic | 1.500 | | Energy Value (kJ) | 281 | 914 |
| Milk Protein | 1.600 | | Energy Value (kcal) | 66 | 216 |
| Corn Oil | 0.600 | | % Energy from Protein | - | 25.37 |
| Flavouring | 0.200 | | % Energy from Carbohydrates | - | 63.89 |
| Emulsifier | 0.150 | | % Energy from Fat | - | 10.83 |
| Colouring | 0.085 | | Trans Fat (g) | 0.006 | 0.02 |
| Vitamin & Mineral Premix | 0.182 | | % Energy from Trans Fat | - | 0.08 |
| Plant Extract (80% steroidal glycosides) | 0.250 | | Linoleic Acid (g) | 0.32 | 1.03 |
| | | | Sodium (mg) | 60 | 200 |
| **TOTAL** | **100.00** | | Cholesterol (mg) | N/A | N/A |

| | | | **Vitamins** | **Per 100ml** | **Per 325ml** |
|---|---|---|---|---|---|
| | | | | | |
| | | | Vitamin A (µg) | 86.00 | 279.50 |
| | | | Vitamin D (µg) | 0.65 | 2.11 |
| | | | Vitamin E (mg) | 1.10 | 3.58 |
| | | | Vitamin C (mg) | 6.50 | 21.13 |
| | | | Thiamin (mg) | 0.23 | 0.75 |
| | | | Riboflavin (mg) | 0.23 | 0.75 |
| | | | Niacin (mg) | 2.70 | 8.78 |
| | | | Vitamin B6 (mg) | 0.28 | 0.91 |
| | | | Folic Acid (µg) | 21.50 | 69.88 |
| | | | Vitamin B12 (µg) | 0.18 | 0.59 |
| | | | Biotin (mg) | 0.02 | 0.05 |
| | | | Pantothenic Acid (mg) | 0.65 | 2.11 |
| | | | | | |

| | | | **Minerals** | **Per 100ml** | **Per 325ml** |
|---|---|---|---|---|---|
| | | | | | |
| | | | Calcium (mg) | 123.00 | 399.75 |
| | | | Phosphorus (mg) | 80.00 | 260.00 |
| | | | Iron (mg) | 1.70 | 5.53 |
| | | | Magnesium (mg) | 20.00 | 65.00 |
| | | | Zinc (mg) | 1.60 | 5.20 |
| | | | Iodine (µg) | 16.20 | 52.65 |
| | | | Potassium (mg) | 154.00 | 500.50 |
| | | | Copper (mg) | 0.15 | 0.49 |
| | | | Selenium (µg) | 7.50 | 24.38 |
| | | | Manganese (mg) | 0.20 | 0.65 |

| | | | | | |
|---|---|---|---|---|---|
| % protein content for the drink = 4.2 % carbohydrate content for the drink = 10.6 % fat content for the drink = 0.8 | | | | | |

The 80% steroidal glycoside had the following specification:

| | | | |
|---|---|---|---|
| Specification item | | Specification Limit | Test method |
| Physical description | | | |
| | Form | Free flowing powder, free from particulate contamination | In-house |
| | Colour | Light Yellow-green | In-house |
| Active Components | | | |
| | Steroidal glycosides | > 80% | HPLC |
| Water content Residual solvents | | < 3% | Karl Fischer |
| | Heptane | <0.5% | |
| | Ethanol | <0.5 % | |
| Particle size Microbiological examination | | < 50 microns | In-house Ph. Eur. |
| count | Total viable aerobic | | |
| | Aerobic | ≤ 10⁴ cfu/g | |
| bacteria | | | |
| | Fungi | ≤ 10² cfu/g | |
| | Enterobacteriaceae | ≤ 10² cfu/g | |
| | *Escherichia coli* | Absent in 1 g | |
| | *Staphylococcus aureus* | Absent in 1 g | |
| | *Salmonella* spp | Absent in 10 g | |

### Example 4

The following appetite reducing acidified dairy drink was prepared:

| **Formulation:** | | | **Nutritional Info:** | | |
|---|---|---|---|---|---|
| **Ingredient** | **% Formula** | | Product weight as consumed: | 90 g | |
| Skim Milk | 6.750 | | Energy density (kJ/g) | 3.71 | |
| Water | To 100% | | **Energy** | **Per 100g wet product** | **Per serving** |
| Sucrose | 5.000 | | | | |
| HM Pectin | 0.450 | | Energy Value (kJ) | 371 | 334 |
| Milk Protein | 1.600 | | Energy Value (kcal) | 89 | 80 |
| Canola oil | 5.000 | | % Energy from Protein | - | 11 |
| Citric acid (50% solution) | 1.250 | | % Energy from Carbohydrates | - | 38 |
| Emulsifier | 0.200 | | % Energy from Fat | - | 51 |
| Flavouring | 0.200 | | | | |
| Acesulfame-K | 0.050 | | | - | |
| Aspartame | 0.050 | | | | |
| Plant Extract (30% steroidal glycosides) | 0.200 | | | | |
| | | | | | |
| **TOTAL** | **100.00** | | | | |

| | | | | | |
|---|---|---|---|---|---|
| % protein content for the drink = 2.4 % carbohydrate content for the drink = 8.4 % fat content for the drink = 5.0 | | | | | |

The 30% extract had the following specification:

| Specification item | Specification limit | Test method |
|---|---|---|
| Physical description | | |
| Form | Powder, free from particulate contamination | In-house |
| Colour | Yellow-green | In-house |
| Active Components | | |
| Steroidal glycosides | > 30% | HPLC |
| Water content | < 5% | Karl Fischer |
| Residual solvents | | |
| Heptane | <1% | |
| Ethanol | <1 % | |
| Particle size | < 100 microns | In-house |
| Microbiological examination | | Ph. Eur. |
| Total viable aerobic count | | |
| Aerobic bacteria | ≤ 10⁴ cfu/g | |
| Fungi | ≤ 10² cfu/g | |
| Enterobacteriaceae | ≤ 10² cfu/g | |
| *Escherichia coli* | Absent in 1 g | |
| *Staphylococcus aureus* | Absent in 1 g | |
| *Salmonella* spp | Absent in 10 g | |

Hoodia gordonii extract water dispersion process:
900 ml demineralised water was heated to 95 ° C in a 1000 ml beaker. The water was agitated using a Silverson mixer set at 5,500 rpm. Then, 8 g sodium stearoyl lactylate was added and the mixture was agitated until the stearoyl lactylate was completely dissolved. The shaft speed of the Silverson was then increased to 6,200 rpm and 8 g Hoodia gordonii extract (obtained according to the process of US 6,376,657) containing about 31% (w/w) steroidal glycosides was added and mixed until it was fully dispersed. The dispersion was then cooled below 40°C by putting the beaker in an ice bath under while continuing mixing at 5000 rpm. Demineralised water was added to a total volume to 1000 ml.

A 4000 ml beaker was filled with 1000 ml of the aqueous Hoodia gordonii dispersion containing 8 g Sodium stearoyl lactylate and 8 g Hoodia gordonii extract and 2200 ml demineralised water. The dispersion was reheated to 75 °C under moderate stirring. The vegetable oil was gently added and mixture was stirred for 2-3 minutes. Then, the skim milk powder, the pectin and the sugars/ sweeteners as a powder blend were added and mixed for 5-8 minutes until all lumps had disappeared. During mixing the citric acid solution was slowly added to set the pH of the drink to 4.0. Finally, demineralised water was added to set the total amount to 4000 g.

### Example 5

Male Sprague-Dawley rats weighing 210 - 250 g were obtained from Harlan Italy s.r.l. (San.Pietro al Natisone (UD), Italy) and individually housed upon arrival. The animals were maintained under a 12:12 light dark cycle (lights off at 18:00) in a temperature and humidity controlled environment (22 ± 2°C/ 55 ± 15% humidity). The animals were fed a standard rat chow and had free access to tap water.

After 1 week of acclimatisation, the animals were randomly assigned to groups of 10 - 15 animals. Each group received by oral gavage either vehicle (0.5% carboxy methyl cellulose (CMC) in water) or Hoodia gordonii extract (30% steroidal glycosides at a dosage of 10, 30 or 60 mg/kg body weight) suspended in vehicle once per day for a minimum of 14 days. Food intake and body weight were measured daily.

Table 2 shows the body weight of rats that received vehicle, 10 mg/kg or *Hoodia gordonii* extract, 30 mg/kg or *Hoodia gordonii* extract or 60 mg/kg or *Hoodia gordonii* extract at day 1 and day 7 of the treatment. Data that were collected are presented as means ± SD.

**TABLE 2**

| | **Body weight (g)** | |
|---|---|---|
| **Treatment** | **Day 1** | **Day 7** |
| Vehicle | 273 ± 9 | 307 ± 12 |
| 10 mg/ kg *Hoodia gordonii* extract | 272 ± 8 | 269 ± 15* |
| 30 mg/ kg *Hoodia gordonii* extract | 269 ± 9 | 241 ± 9* |
| 60 mg/ kg *Hoodia gordonii* extract | 271 ± 9 | 236 ± 11* |

At day 7 of the treatment, the mean body weights of rats receiving 10, 30 and 60 mg/kg *Hoodia gordonii* extract was significantly different from rats receiving vehicle (p < 0.01, Dunnett's test - denoted by * in Table 1).

Table 3 shows the daily food intake of rats that received vehicle, 10 mg/kg or *Hoodia* gordonii extract, 30 mg/kg of Hoodia gordonii extract or 60 mg/kg of Hoodia gordonii extract at day 7. Data are presented as means ± SD.

**TABLE 3**

| **Treatment** | **Daily Food Intake at Day 7 (g/ animal/ day)** | **Decrease in Food Intake at Day 7 (% of control)** |
|---|---|---|
| Vehicle | 26.7 ± 2.1 | 0 |
| 10 mg/ kg *Hoodia gordonii* extract/ day | 15.9 ± 5.2 | 40 ± 19 |
| 30 mg/ kg *Hoodia gordonii* extract/ day | 7.6 ± 2.5 | 72 ± 9 |
| 60 mg/ kg *Hoodia gordonii* extract/ day | 5.9 ± 1.4 | 78 ± 5 |

### Example 6

The data from Example 6 are used to show the effects of simultaneous consumption of a nourishing amount of energy and steroidal glycoside on energy intake in humans, assuming a person of 70 kg with an average daily energy consumption of 10,000 kJ and assuming 20% reduction in energy intake from 350 mg steroidal glycosides, 40% reduction in energy intake from 700 mg steroidal glycosides, 60% reduction in energy intake from 1050 mg steroidal glycosides, 72% reduction in energy intake from 2100 mg steroidal glycosides and 78% reduction in energy intake from 4200 mg steroidal glycosides. Table 4 shows the decrease in energy intake in such a typical person after consuming increasing numbers of unit servings. Product A (outside the scope of the invention) contains a very low amount of energy, i.e. 100 kJ per unit serving and the total daily energy intake falls very rapidly with the increasing number of servings, below the levels that are desirable. In Product 1 (within the scope of the invention), the amount of steroidal glycoside in the unit serving is balanced with a nourishing amount of energy (i.e. 800 kJ) This energy adds to the total daily energy intake of the person consuming the unit servings, resulting in a 12% to 36% reduction of energy intake over a wide range of unit servings consumed per day. Product B (outside the scope of the invention) contains too high amount energy per unit serving, with the result that the reduction in energy intake caused by steroidal glycoside is reversed by the energy of the unit servings as shown and no reduction in daily energy intake is observed. As shown, the right balance between the amount of the steroidal glycoside and the amount of energy in the unit serving determines the maximum decrease in energy intake.

As shown in this example, the reduction in energy intake is 1200 to 3600 kJ over a wide range of unit servings consumed per day.

**TABLE 4**

| | **Total Daily Energy Intake** | | | |
|---|---|---|---|---|
| | **(kJ/ day)** | | | |
| **Number Unit Serving s per Day** | Product 1 350 mg steroidal glycoside and 800 kJ energy (1:2.3 ratio) | Product 2 350 mg steroidal glycoside and 2000 kJ energy (1:5.7ratio) | Product A 350 mg steroidal glycoside and 100 kJ energy (3.5:1 ratio) | Product B 350 mg steroidal glycoside and 2600 kJ energy (1:7.4 ratio) |
| 1 | 8,800 | 10,000 | 8,100 | 10,600 |
| 2 | 7,600 | 10,000 | 6,200 | 11,200 |
| 3 | 6,400 | 10,000 | 4,300 | 11,800 |
| 6 | 7,600 | 14,800 | 3,400 | 14,800 |
| 12 | 11,800 | 26,200 | 3,400 | 33,400 |

## Claims

1. An edible appetite suppressant unit serving product in the form of a bar having a weight from 20 to 100 g, or a drink having a volume from 80 to 600ml, the product comprising:
(a) from 50 to 2000 milligrams of a steroidal glycoside obtainable from plants of the *Hoodia* genus;
(b) from 200 to 2, 000 kilojoules total energy;
(c) the steroidal glycoside to total energy ratio of from 1 : 0.5 to 1 : 10 mg/kJ;
(d) an essential mineral selected from the group consisting of phosphorus, iron, zinc, copper, selenium, magnesium, manganese, molybdenum, chromium and mixtures thereof.

2. The product of claim 1 comprising from 70 to 1500 mg of the steroidal glycoside.

3. The product according to any one of the preceding claims comprising from 90 to 1100 mg of the steroidal glycoside.

4. The product according to any one of the preceding claims wherein the steroidal glycoside is obtainable from the plants selected from the group consisting of *Trichocaulon piliferum, Trichocaulon officinale, Hoodia currorii, Hoodia gordonii, Hoodia lugardii* and mixtures thereof.

5. The product according to any one of the preceding claims wherein the steroidal glycoside is obtainable from *Hoodia gordonii.*

6. The product according to any one of the preceding claims wherein the steroidal glycoside is incorporated into the product in the form of a concentrated preparation obtainable from plants.

7. The product according to claim 6 wherein the concentrated preparation comprises at least 10% of the steroidal glycoside, by weight of the concentrated preparation.

8. The product according to any one of the preceding claims wherein the steroidal glycoside is incorporated into the product in the form of an extract obtainable from plants.

9. The product according to any one of the preceding claims wherein the steroidal glycoside comprises a deoxy or a di-deoxy glycoside.

10. The product according to any one of the preceding claims, wherein the steroidal glycoside has the general Formula (1): 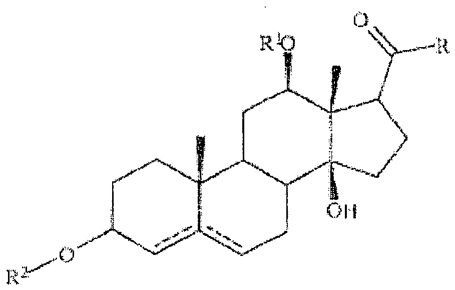 wherein
R = alkyl;
R¹ is selected from the group consisting of H, alkyl, tiglyol, benzoyl and an organic ester group;
R² is selected from the group consisting of H, or one or more 6-deoxy carbohydrates, or one or more 2,6-dideoxy carbohydrates, glucose radical, and mixtures thereof; and wherein the broken lines indicate the optional presence of a further bond between carbon atoms C4 and C5 or between carbon atoms C5 and C6.

11. The product according to any one of the preceding claims, wherein essential minerals are selected from the group consisting of iron, phosphorus and zinc.

12. The product according to any one of the preceding claims, wherein the steroidal glycoside comprises a compound of Formula (2):

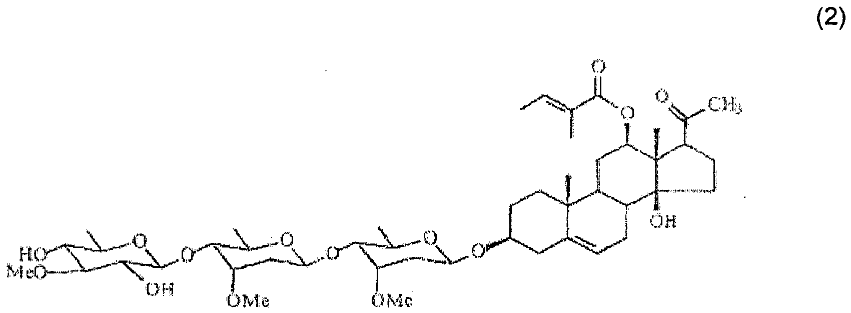

13. The product according to any one of the preceding claims wherein the essential mineral is iron.

14. The product of claim 13 wherein the ratio of steroidal glycoside to iron is in the range of from 25:1 to 500:1 (mg/mg).

15. The product according to any one of the preceding claims, wherein the product contains from 250 kJ to 2,000 kJ total energy.

16. The product according to any one of the preceding claims wherein the ratio of steroidal glycoside to the total amount of energy is in the range of from 1:0.7 mg/kJ to 1:8 mg/kJ.

17. The product according to any one of the preceding claims, wherein from 3% to 50% of the total energy is from protein.

18. The product according to any one of the preceding claims in the form of the unit serving drink comprising in addition to the steroidal glycoside:
from 75% to 95 wt% moisture;
from 0.5% to 10 wt% protein;
from 0.5% to 6 wt% fat;
from 3% to 20 wt% carbohydrate;
and up to 8 wt% optional ingredients.

19. The product according to any one of the preceding claims in the form of the unit serving bar comprising in addition to the steroidal glycoside:
from 3 to 30 wt% moisture
from 3 to 30 wt% protein
from 3 to 30 wt% fat
from 35 to 80 wt% carbohydrate
and up to 12 wt% optional ingredients.

20. The product according to any one of the preceding claims wherein the weight ratio of protein to steroidal glycoside is in the range of from 0.5:1 to 200:1.

21. A non-therapeutic method of suppressing appetite in a human while also providing sufficient nourishment, the method comprising having the human ingest the product according to claim 1.

22. The method according to claim 21, the method comprising ingesting from 1 to 5 of the products per day.

23. The method according to claim 21, the method comprising ingesting from 1 to 3 of the products per day.

24. The method according to claim 21 achieving daily energy intake reductions in a human adult of from 750 to 4,500 kJ per day.

25. The method according to claim 21, wherein the product is consumed for at least 14 days.

26. The method according to claim 21 wherein the total daily energy intake provided by the unit serving products is from 200 kJ to 5,000 kJ.

27. The product according to claim 1 for use in a method of controlling obesity in a human while also providing sufficient nourishment, wherein the human ingests the product.

28. The product for use according to claim 27, the method comprising ingesting from 1 to 5 of the products per day.

29. The product for use according to claim 27, the method comprising ingesting from 1 to 3 of the products per day.

30. The product for use according to claim 27, whereby the method achieves daily energy intake reductions in a human adult of from 750 to 4,500 kJ per day.

31. The product for use according to claim 27, wherein the product is consumed for at least 14 days.

32. The product for use according to claim 27, wherein the total daily energy intake provided by the unit serving products is from 200 kJ to 5,000 kJ.

## Patentansprüche

1. Essbares Appetitzügler-Produkt als Einheitsportion in Form eines Riegels mit einem Gewicht von 20 bis 100 g oder eines Getränks mit einem Volumen von 80 bis 600 ml, wobei das Produkt:
(a) 50 bis 2000 Milligramm eines Steroidglykosids, erhältlich aus Pflanzen der Gattung *Hoodia;*
(b) 200 bis 2.000 Kilojoule Gesamtenergie;
(c) ein Steroidglykosid-zu-Gesamtenergie-Verhältnis von 1 : 0,5 bis 1 : 10 mg/kJ;
(d) ein essentielles Mineral, ausgewählt aus der Gruppe, bestehend aus Phosphor, Eisen, Zink, Kupfer, Selen, Magnesium, Mangan, Molybdän, Chrom und Gemischen davon, umfasst.

2. Produkt nach Anspruch 1, umfassend 70 bis 1500 mg des Steroidglykosids.

3. Produkt nach einem der vorhergehenden Ansprüche, umfassend 90 bis 1100 mg des Steroidglykosids.

4. Produkt nach einem der vorhergehenden Ansprüche, wobei das Steroidglykosid aus Pflanzen, ausgewählt aus der Gruppe, bestehend aus *Trichocaulon piliferum*, *Trichocaulon officinale, Hoodia currorii*, *Hoodia gordonii, Hoodia lugardii* und Gemischen davon, erhältlich ist.

5. Produkt nach einem der vorhergehenden Ansprüche, wobei das Steroidglykosid aus *Hoodia gordonii* erhältlich ist.

6. Produkt nach einem der vorhergehenden Ansprüche, wobei das Steroidglykosid in das Produkt in Form eines konzentrierten Präparats, das aus Pflanzen erhältlich ist, eingebracht wird.

7. Produkt nach Anspruch 6, wobei das konzentrierte Präparat mindestens 10 % des Steroidglykosids, bezogen auf das Gewicht des konzentrierten Präparats, umfasst.

8. Produkt nach einem der vorhergehenden Ansprüche, wobei das Steroidglykosid in das Produkt in Form eines aus Pflanzen erhältlichen Extrakts eingebracht wird.

9. Produkt nach einem der vorhergehenden Ansprüche, wobei das Steroidglykosid ein Desoxy- oder ein Didesoxyglykosid umfasst.

10. Produkt nach einem der vorhergehenden Ansprüche, wobei das Steroidglykosid die allgemeine Formel (1) hat: 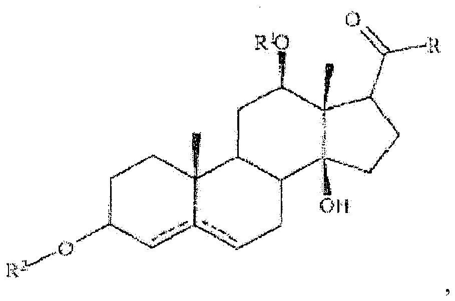 worin
R = Alkyl;
R¹ ausgewählt ist aus der Gruppe, bestehend aus H, Alkyl, Tiglyol, Benzoyl und einer organischen Estergruppe;
R² ausgewählt ist aus der Gruppe, bestehend aus H oder einem oder mehreren 6-Desoxy-kohlenhydraten oder einem oder mehreren 2,6-Didesoxykohlenhydraten, einem Glukoserest und Gemischen davon; und wobei die unterbrochenen Linien die optionale Gegenwart einer weiteren Bindung zwischen den Kohlenstoffatomen C4 und C5 oder zwischen den Kohlenstoffatomen C5 und C6 anzeigt.

11. Produkt nach einem der vorhergehenden Ansprüche, wobei die essentiellen Minerale ausgewählt sind aus der Gruppe, bestehend aus Eisen, Phosphor und Zink.

12. Produkt nach einem der vorhergehenden Ansprüche, wobei das Steroidglykosid eine Verbindung der Formel (2) umfasst:

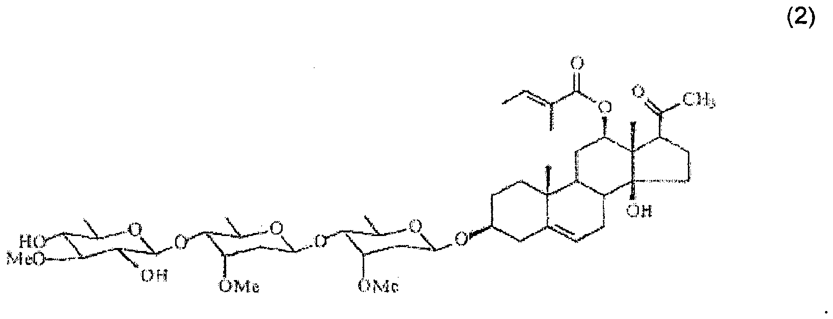

13. Produkt nach einem der vorhergehenden Ansprüche, wobei das essentielle Mineral Eisen ist.

14. Produkt nach Anspruch 13, wobei das Verhältnis von Steroidglykosid zu Eisen im Bereich von 25 : 1 bis 500 : 1 (mg/mg) liegt.

15. Produkt nach einem der vorhergehenden Ansprüche, wobei das Produkt 250 kJ bis 2.000 kJ Gesamtenergie enthält.

16. Produkt nach einem der vorhergehenden Ansprüche, wobei das Verhältnis von Steroidglykosid zu der Gesamtmenge an Energie im Bereich von 1 : 0,7 mg/kJ bis 1 : 8 mg/kJ liegt.

17. Produkt nach einem der vorhergehenden Ansprüche, wobei 3 % bis 50 % der Gesamtenergie aus Protein stammen.

18. Produkt nach einem der vorhergehenden Ansprüche in Form des Getränks als Einheitsportion, umfassend neben dem Steroidglykosid:
75 bis 95 Gew.-% Feuchtigkeit;
0,5 bis 10 Gew.-% Protein;
0,5 bis 6 Gew.-% Fett;
3 bis 20 Gew.-% Kohlenhydrat
und bis zu 8 Gew.-% optionale Inhaltsstoffe.

19. Produkt nach einem der vorhergehenden Ansprüche in Form des Riegels als Einheitsportion, umfassend neben dem Steroidglykosid:
3 bis 30 Gew.-% Feuchtigkeit;
3 bis 30 Gew.-% Protein;
3 bis 30 Gew.-% Fett;
35 bis 80 Gew.-% Kohlenhydrat
und bis zu 12 Gew.-% optionale Inhaltsstoffe.

20. Produkt nach einem der vorhergehenden Ansprüche, wobei das Gewichtsverhältnis von Protein zu Steroidglykosid im Bereich von 0,5 : 1 bis 200 : 1 liegt.

21. Nicht therapeutisches Verfahren zum Zügeln des Appetits bei einem Menschen, das ebenso ausreichend Nahrung bereitstellt, wobei in dem Verfahren der Mensch das Produkt nach Anspruch 1 aufnimmt.

22. Verfahren nach Anspruch 21, wobei das Verfahren die Aufnahme von 1 bis 5 Produkten pro Tag umfasst.

23. Verfahren nach Anspruch 21, wobei das Verfahren die Aufnahme von 1 bis 3 Produkten pro Tag umfasst.

24. Verfahren nach Anspruch 21, das eine Verringerung der täglichen Energieaufnahme bei einem erwachsenen Menschen von 750 bis 4.500 kJ pro Tag erreicht.

25. Verfahren nach Anspruch 21, wobei das Produkt für mindestens 14 Tage konsumiert wird.

26. Verfahren nach Anspruch 21, wobei die von den Produkten als Einheitsportion bereitgestellte tägliche Gesamtenergieaufnahme 200 kJ bis 5.000 kJ beträgt.

27. Produkt nach Anspruch 1 zur Verwendung in einem Verfahren zur Kontrolle von Fettleibigkeit beim Menschen, das ebenso ausreichend Nahrung bereitstellt, wobei der Mensch das Produkt aufnimmt.

28. Produkt zur Verwendung nach Anspruch 27, wobei das Verfahren die Aufnahme von 1 bis 5 Produkten pro Tag umfasst.

29. Produkt zur Verwendung nach Anspruch 27, wobei das Verfahren die Aufnahme von 1 bis 3 Produkten pro Tag umfasst.

30. Produkt zur Verwendung nach Anspruch 27, wobei das Verfahren eine Verringerung der täglichen Energieaufnahme bei einem erwachsenen Menschen von 750 bis 4.500 kJ pro Tag erreicht.

31. Produkt zur Verwendung nach Anspruch 27, wobei das Produkt für mindestens 14 Tage konsumiert wird.

32. Produkt zur Verwendung nach Anspruch 27, wobei die von den Produkten als Einheitsportion bereitgestellte tägliche Gesamtenergieaufnahme 200 kJ bis 5.000 kJ beträgt.

## Revendications

1. Produit anorexigène comestible en dose unitaire, sous la forme d'une barre ayant un poids de 20 g à 100 g ou d'une boisson ayant un volume de 80 ml à 600 ml, le produit comprenant :
(a) de 50 à 2000 milligrammes d'un glycoside stéroïdien pouvant être obtenu à partir de plantes du genre *Hoodia ;*
(b) de 200 à 2000 kilojoules d'énergie totale ;
(c) le rapport glycoside stéroïdien sur énergie totale de 1/05 à 1/10 mg/kJ ;
(d) un minéral essentiel choisi dans le groupe constitué du phosphore, du fer, du zinc, du cuivre, du sélénium, du magnésium, du manganèse, du molybdène, du chrome et de leurs mélanges.

2. Produit selon la revendication 1, comprenant de 70 mg à 1500 mg du glycoside stéroïdien.

3. Produit selon l'une quelconque des revendications précédentes, comprenant de 90 mg à 1100 mg du glycoside stéroïdien.

4. Produit selon l'une quelconque des revendications précédentes, dans lequel le glycoside stéroïdien peut être obtenu à partir de plantes choisies dans le groupe constitué de *Trichocaulon piliferum, Trichocaulon officinale, Hoodia currorii, Hoodia gordonii, Hoodia lugardii* et leurs mélanges.

5. Produit selon l'une quelconque des revendications précédentes, dans lequel le glycoside stéroïdien peut être obtenu à partir de *Hoodia gordonii.*

6. Produit selon l'une quelconque des revendications précédentes, dans lequel le glycoside stéroïdien est incorporé dans le produit sous la forme d'une préparation concentrée pouvant être obtenue à partir de plantes.

7. Produit selon la revendication 6, dans lequel la préparation concentrée comprend au moins 10 % du glycoside stéroïdien, en poids de la préparation concentrée.

8. Produit selon l'une quelconque des revendications précédentes, dans lequel le glycoside stéroïdien est incorporé dans le produit sous la forme d'un extrait pouvant être obtenu à partir de plantes.

9. Produit selon l'une quelconque des revendications précédentes, dans lequel le glycoside stéroïdien comprend un désoxy- ou di-désoxy-glycoside.

10. Produit selon l'une quelconque des revendications précédentes, dans lequel le glycoside stéroïdien possède la formule générale (1) : 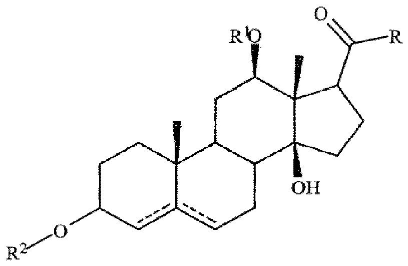 dans laquelle
R = alkyle ;
R¹ est choisi dans le groupe constitué de H, d'un alkyle, d'un tiglyol, d'un benzoyle et d'un groupe ester organique ;
R² est choisi dans le groupe constitué de H, ou d'un ou de plusieurs 6-désoxy-glucides, ou d'un ou de plusieurs 2,6-didésoxy-glucides, d'un radical glucose et de leurs mélanges ; et dans laquelle les lignes pointillées indiquent la présence optionnelle d'une liaison supplémentaire entre les atomes de carbone C4 et C5 ou entre les atomes de carbone C5 et C6.

11. Produit selon l'une quelconque des revendications précédentes, dans lequel les minéraux essentiels sont choisis dans le groupe constitué du fer, du phosphore et du zinc.

12. Produit selon l'une quelconque des revendications précédentes, dans lequel le glycoside stéroïdien comprend un composé de formule (2) :

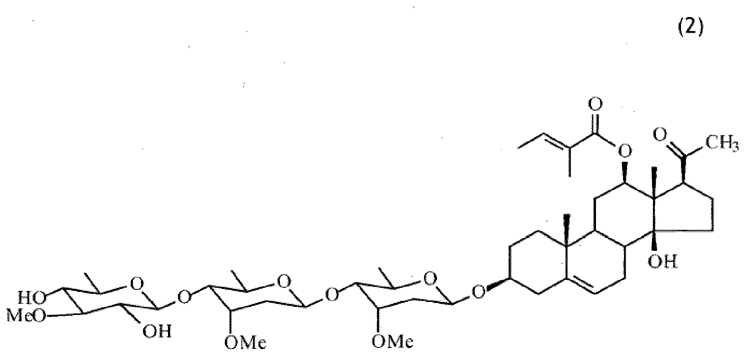

13. Produit selon l'une quelconque des revendications précédentes, dans lequel le minéral essentiel est le fer.

14. Produit selon la revendication 13, dans lequel le rapport glycoside stéroïdien sur fer est situé dans la plage allant de 25/1 à 500/1 (mg/mg).

15. Produit selon l'une quelconque des revendications précédentes, dans lequel le produit contient 250 kJ à 2000 kJ d'énergie totale.

16. Produit selon l'une quelconque des revendications précédentes, dans lequel le rapport glycoside stéroïdien sur la quantité totale d'énergie est situé dans la plage allant de 1/0,7 mg/kJ à 1/8 mg/kJ.

17. Produit selon l'une quelconque des revendications précédentes, dans lequel de 3 % à 50 % de l'énergie totale proviennent des protéines.

18. Produit selon l'une quelconque des revendications précédentes, sous la forme de la boisson en dose unitaire comprenant en plus du glycoside stéroïdien :
de 75 % à 95 % en poids d'humidité ;
de 0,5 % à 10 % en poids de protéines ;
de 0,5 % à 6 % en poids de matières grasses ;
de 3 % à 20 % en poids de glucides ;
et jusqu'à 8 % en poids d'ingrédients optionnels.

19. Produit selon l'une quelconque des revendications précédentes, sous la forme de la barre en dose unitaire comprenant en plus du glycoside stéroïdien :
de 3 % à 30 % en poids d'humidité ;
de 3 % à 30 % en poids de protéines ;
de 3 % à 30 % en poids de matières grasses ;
de 35 % à 80 % en poids de glucides ;
et jusqu'à 12 % en poids d'ingrédients optionnels.

20. Produit selon l'une quelconque des revendications précédentes, dans lequel le rapport pondéral protéines sur glycoside stéroïdien est situé dans la plage allant de 0,5/1 à 200/1.

21. Procédé non thérapeutique de suppression de l'appétit chez un humain tout en fournissant également une alimentation suffisante, le procédé comprenant l'ingestion par l'humain du produit selon la revendication 1.

22. Procédé selon la revendication 21, le procédé comprenant l'ingestion du produit 1 à 5 fois par jour.

23. Procédé selon la revendication 21, le procédé comprenant l'ingestion du produit 1 à 3 fois par jour.

24. Procédé selon la revendication 21, permettant d'atteindre une réduction de prise énergétique quotidienne chez un adulte humain de 750 kJ à 4500 kJ par jour.

25. Procédé selon la revendication 21, dans lequel le produit est consommé pendant au moins 14 jours.

26. Procédé selon la revendication 21, dans lequel la prise énergétique quotidienne totale fournie par les produits en dose unitaire est de 200 kJ à 5000 kJ.

27. Produit selon la revendication 1, pour son utilisation dans un procédé de lutte contre l'obésité chez un humain tout en fournissant également une alimentation suffisante, dans lequel l'humain ingère le produit.

28. Produit pour son utilisation selon la revendication 27, le procédé comprenant l'ingestion du produit 1 à 5 fois par jour.

29. Produit pour son utilisation selon la revendication 27, le procédé comprenant l'ingestion du produit 1 à 3 fois par jour.

30. Produit pour son utilisation selon la revendication 27, moyennent quoi le procédé permet d'atteindre une réduction de prise énergétique quotidienne chez un adulte humain de 750 kJ à 4500 kJ par jour.

31. Produit pour son utilisation selon la revendication 27, dans lequel le produit est consommé pendant au moins 14 jours.

32. Produit pour son utilisation selon la revendication 27, dans lequel la prise énergétique quotidienne totale fournie par les produits en dose unitaire est de 200 kJ à 5000 kJ.
